# EUROPEAN PATENT SPECIFICATION

(11) **EP 0 597 446 B1**
(45) Date of publication and mention of the grant of the patent: **22.04.1998**
(21) Application number: 93118151.5
(22) Date of filing: 09.11.1993
(51) Int. Cl.: B65D 75/58, A61F 13/20, B31F 1/00

(54) **Tampon pack**
Tamponpackung
Tampon hygiénique emballé

(30) Priority: 09.11.1992 DE 4237795
(43) Date of publication of application: 18.05.1994
(73) Proprietor: McNEIL-PPC, INC., Milltown New Jersey 08850 (US)
(72) Inventor: Zielke, Helmut, Dr., D-25587 Münsterdorf (DE); Schoelling, Hans-Werner, D-58256 Ennepetal (DE); Leutwyler, Robert, CH-8113 Boppelsen (CH)
(74) Representative: Groening, Hans Wilhelm, Dipl.-Ing.

(56) References cited:
- EP-A- 0 395 249
- EP-A- 0 445 565
- CH-A- 512 378
- DE-U- 9 107 954
- GB-A- 2 208 622
- US-A- 2 236 366
- US-A- 2 546 698
- US-A- 2 769 496
- US-A- 3 014 631
- US-A- 3 978 752
- US-A- 3 978 753
- US-A- 4 050 362
- US-A- 4 557 385
- US-A- 4 982 845
- US-A- 5 133 457

## Description

The invention relates to an individual, substantially airtight pack containing a digital tampon for feminine hygiene according to the preamble of claim 1. Such a pack is known from DE-A-19 62 733.

DE-A-19 62 733 discloses an individual, substantially airtight pack containing a digital tampon for feminine hygiene of which the rear end is provided with a recovery tape. Said pack comprises packaging material enclosing said individual tampon and having means arranged around the circumference of the individual tampon pack for opening the package. Cellophane is used as packaging material and coated with glue. Longitudinal slits are made in the vicinity of the glue-coating points, before the packaging sleeve is wound up and closed at one end for the subsequent reception of the tampon. A handle for opening the pack is provided by cutting through the packaging material just in front of the rear end of the two longitudinal slits located next to one another.

US-A-4,982,845 discloses a flexible, resealable packaging enclosure utilizing a flap for resealing an aperture through which the products are dispensed. The known packaging enclosure is directed to the object to reduce any risk of contamination of the products contained within the enclosure, even during lengthy storage, to resist premature opening from forces exerted on the enclosure before use and to provide easy access to the contents of the enclosure in the hands of the consumer. This is realised by a resealable enclosure comprising a cover having a top panel which has side edges and a central portion intermediate said side edges. Means are provided to define a tear path in said top panel, said tear path having a width which extends across said central portion of said top panel to form an aperture through which products may be extracted from the enclosure. Said defining means have a weaker portion where said tear path intersects said central portion of said top panel and stronger portions on either side of said weaker portion proximate said side edges. Said stronger portions are more resistant than said weaker portions to forces tending to separate said panel along said tear path. A closure tab has a first end permantently secured to said top panel on one side of said tear path. A second end of said tap has an adhesive coating facilitating releasable attachment of said second end to said top panel on the opposite side of said tear path to close said aperture. Said second end attaches to said top panel in a region of said weaker portion. Perforations of said stronger portion are spaced apart from one another by a distance greater than a spacing between said perforations of said weaker portions. Furthermore, the perforations of the weaker portion can have a length greater than a length of the perforations comprising the stronger portions. Finally, the top panel of the enclosure has a thickness and said stronger portions and weaker portions of said defining means comprise a groove-like indentation formed in said top panel. Said stronger portions of said indentation are shallower in the direction of said thickness than said weaker portion of said indentation.

CH-A-512 378 discloses a package for food products, particularly fatty bodies like butter. The packaging material for this package consists of a lamination of paper and a foil of aluminum. Packages of this type seal the content to avoid an oxidation of the content and to retent its odor. Once the package is open, it will lose this characteristic so that the food in the package might be oxidized and the content might lose its flavour. As to solve this problem, this document proposes the provision of a plurality of parallel lines of less resistance in said combination of paper/aluminum packaging material to delimit the open zones of the package produced by the destruction of each of said plurality of weakness lines, so that only a determined portion of the food product will be usable for consumption whilst the main portion of the food is still protected by the package.

US-A-5,133,457 discloses a tampon applicator assembly wherein a protective wrap which initially packages the tampon applicator provides a covering for disposal of the spent applicator. The tampon applicator wrap is opened at a pre-determined location such that a discrete portion remains which is significantly longer than the applicator barrel member. After tampon ejection, with the plunger telescopingly located within the barrel, said unit is then inserted within the discrete wrap portion, which includes a substantial length extending beyond the re-inserted applicator. Said substantial length is then twisted and tucked into the open end of the applicator to maintain closure of the wrap for applicator disposal.

DE-U-91 07 954 discloses a packaging bag for hygiene articles like diapers being positioned in the back under a certain compression. Said bag has regions being surrounded by weakening lines which form a tap to open only a small portion of the package. The weakening line can be made by thinning of the material, but preferably by perforation.

US-A-2,236,366 discloses a machine having a structure for performing a scoring or creasing operation. To this end, a shaft has thereon a disk having a circumferential groove in its outer cylindrical surface to cooperate with a projecting V-shaped creasing edge on the periphery of a disk secured on the shaft. For scoring a web passing through the device, the shaft has a disk secured thereon presenting a cylindrical outer surface to cooperate with a peripheral scoring edge on the outer periphery of a disk secured against rotation on the shaft. The diameter of the several disk are so controlled that the edge will enter the notch for creasing the web or sheet and the edge will be spaced from the cylindrical surface at a distance materially less than the thickness of the web for scoring the web or sheet.

In packs for daily commodities, very high demands are made on the closure which has to close the pack securely and, if desired, also in an airtight manner for a relatively long period of time in order to prevent the contents of the pack from becoming contaminated. Corresponding demands are also to be made of the packaging material itself, cellophane above all having proved appropriate. On the other hand, since a rapid and reliable opening of the pack is a necessary precondition in packs of this type for mass-produced daily commodities of mass consumption, such packs have for many years been provided with perforations at the point where opening is desired. Such perforations allow, as a result of the inflow of air, contamination and, furthermore, as a result of the inflow of water vapour contained in the air, expansion of a pack content which consists at least partially of hydrophilic material. Moreover, perforations occasionally have some weakness in respect of bending stresses. For the reasons mentioned, packaging materials allowing an airtight welding of the packs have increasingly been used. In conjunction with this, the provision of notch lines and tear-open strips for opening the packs has become known. The production of these packs necessitates the increased use of material and a higher outlay in manufacturing terms. In addition, the opening of packs of this type is often difficult and time-consuming, because the tear-open strip is not lifted off from the surface of the pack, frequently even adheres to the pack and therefore cannot always be located or detached immediately by the consumer.

The opening of a cellophane pack along a line of weakness consisting of perforations entails a disturbing noise which often resembles a crack. In such packs for daily commodities, such as semi-luxuries, but also articles of hygiene, such as, for example, tampons for feminine hygiene, this generation of noise is undesirable and often embarrassing.

The object on which the invention is based is, therefore, to improve a packaging material of the abovementioned known generic type, in such a way that as small a quantity as possible of packaging material is needed for a pack for mass-produced articles, such as articles of hygiene or semi-luxuries, such as sweets or the like. At the same time, the pack is to be capable of being opened easily as silently and as discreetly as possible and in a manner free of residues of packaging material, but is nevertheless to be relatively stable in respect of bending and impact stresses.

The present invention achieves this object by the features disclosed in the characterizing portion of claim 1.

Subclaims 2 to 41 disclose additional improving features of the invention. Accordingly, the invention provides a line of weakness having weak points of varying material strength, so that the line of weakness can be destroyed essentially silently and in a manner free of residues of packaging material. Zones of reduced strength can be provided at each individual weak point. These zones of reduced strength can be so designed that each weak point is embossed in and/or transversely to the direction of the line of weakness first lightly, then heavily and subsequently lightly. An especially silent and residue-free destruction of the line of weakness can be achieved if the zones of reduced strength are designed to extend continuously and gradually into one another. At the same time, the weak points can be provided both on the top side and on the underside of the packaging material.

In those instances in which packaging material is provided with at least one line of weakness, the weak points of which are separated by webs, according to a further embodiment of the invention a varying size of the individual webs between the weak points can be provided. A continuous variation of the web size in one direction of the line of weakness is to be preferred for this. If appropriate there can also be provided a plurality of mutually adjoining length portions of the line of weakness having webs, the size of which increases or decreases continuously in one direction of the line of weakness. The weak points can be arranged identically or in varying length and/or in the form of patterns. According to the invention, in an especially advantageous version of the line of weakness, its weak points are made by embossing. However, if there are less strict requirements as to the airtightness and/or hygiene of a pack and depending on the type of packaging material and of the pressure exerted in order to make the line of weakness, the weak points can also be designed as webs with varying dimensions between perforations of the packaging material.

Furthermore, the packaging material can be provided with embossings which improve the grip or the handling and/or the properties of a pack produced from the packaging material. For this purpose, the packaging material can also be coated. If it is coated on the outside, the outer layer can consist of a roughness lacquer which likewise serves for improving the handling and airtightness of the material. However, the packaging material can also additionally have an inner coating which serves for the airtight inclusion of the pack contents and/or for the easier extraction of the pack content. Furthermore, the said coatings also additionally contribute to the damping of noise when the pack is being opened.

According to the invention, cellophane and biologically degradable plastic foils are preferred as packaging materials. At the same time, the packaging material can have a thickness of 10 to 60 microns; a thickness of at least 10 microns is preferred, but a thickness which amounts at most to 20 microns is the most preferable.

The individual pack for tampons for feminine hygiene according to the present invention allows the desired discreet, silent and residue-free opening of the pack, whilst the tampon in the pack is protected from contamination and from the inflow of water vapour until the latter is opened.

The invention is described in detail below by means of the diagrammatic drawings of numerous exemplary embodiments of the packaging material and of apparatuses and processes suitable for making the lines of weakness in the packaging material. In the drawings:
- Figure 1: shows a perspective representation of a portion of packaging material having a line of weakness suitable for carrying out the invention;
- Figure 2: shows a longitudinal section along the sectional line II-II in Figure 1;
- Figure 3: shows a cross-section along the sectional line III-III in Figure 1;
- Figures 4 to 9: show longitudinal sections through lines of weakness of a packaging material having weak points of varying longitudinal profile;
- Figures 10(a) to 10(h): show a graphical representation of individual packs of tampons for feminine hygiene, having lines of weakness consisting of varying patterns of weakness;
- Figure 11: shows a longitudinal section through a packaging material having an outer layer consisting of roughness lacquer;
- Figure 12: shows an individual pack of a tampon for feminine hygiene consisting of the packaging material according to Figure 11;
- Figures 13 and 14: show a cross-section through the packaging material having mechanical embossings;
- Figures 15(a) to 15(f): show a graphical representation of individual packs of tampons for feminine hygiene, having various mechanical embossed patterns;
- Figure 16: shows a part view of a first embodiment of a weakening disc;
- Figure 17: shows a weakening tooth of Figure 16 on a larger scale;
- Figure 18: a partially cutaway view of a cross-section along the sectional line XVIII-XVIII in Figure 17 with a counterroller;
- Figure 19: shows a graphical view of the production of a line of weakness in a packaging material by means of a weakening disc according to Figures 17 and 18;
- Figure 20: shows a part view of a further embodiment of a weakening disc;
- Figure 21: shows the detail X of the weakening disc in Figure 20 on an enlarged scale;
- Figure 22: shows the representation according to Figures 20 and 21 on an even larger scale, a differing weakening of the packaging material being shown diagrammatically by differing weakening teeth;
- Figure 23: shows a part view of a second embodiment of a weakening disc;
- Figure 24: shows a detail X in Figure 23 on a larger scale;
- Figure 25: shows a part view of a further embodiment of a weakening disc;
- Figure 26: shows a detail X in Figure 25 on a larger scale;
- Figure 27: shows a part view of a modified embodiment of a weakening tooth;
- Figure 28: shows a part view of a further embodiment of a weakening disc;
- Figure 29: shows a detail X in Figure 28 on a larger scale;
- Figure 30: shows a cross-section along the sectional line XXX-XXX in Figure 28;
- Figure 31: shows a part view of a further embodiment of a weakening disc;
- Figure 32: shows a top view of oblique weakening-tooth tips according to the arrow A in Figure 31; and
- Figure 33: shows a detail X in Figure 31 on a larger scale.

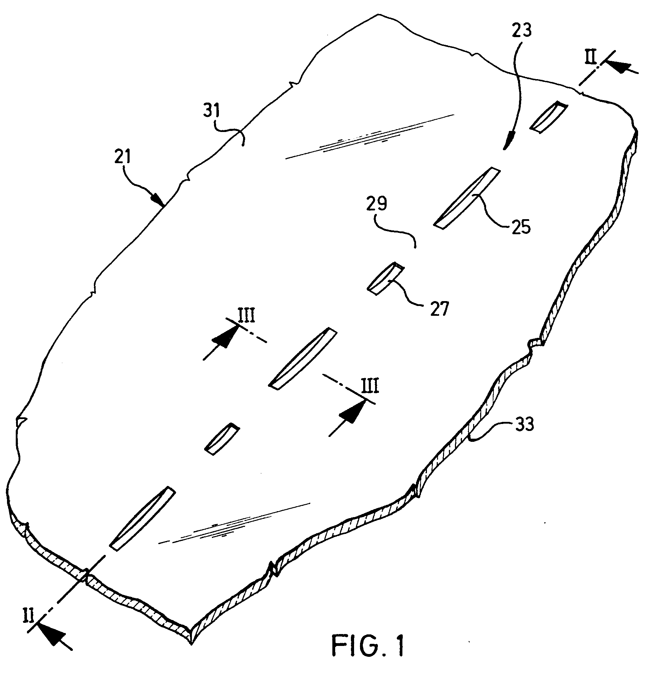

Figure 1 shows an especially preferred embodiment of a packaging material 21 having a line of weakness 23. Line of weakness 23 consists of a plurality of weak points 25, 27 which are arranged alternately in succession at equal intervals formed by webs 29. The weak points 25 are designed differently from the weak points 27. It is evident that each weak point 25 extends over a greater length than each of the weak points 27. However, the width of the weak points 25, 27 is made approximately equal. If required, however, the width of one or more weak points can also be chosen so as to vary. It is clear that the webs 29 between the weak points 25, 27 make the line of weakness 23 less sensitive to bending stresses transversely to the longitudinal direction of the line of weakness 23. At the same time, the weak points 25, 27, at which the packaging material 21 is only embossed, but not cut through, ensure an airtight, hygienically satisfactory packaging of articles until the largely silent destruction of the line of weakness 23 for the purpose of opening a pack consisting of the packaging material.

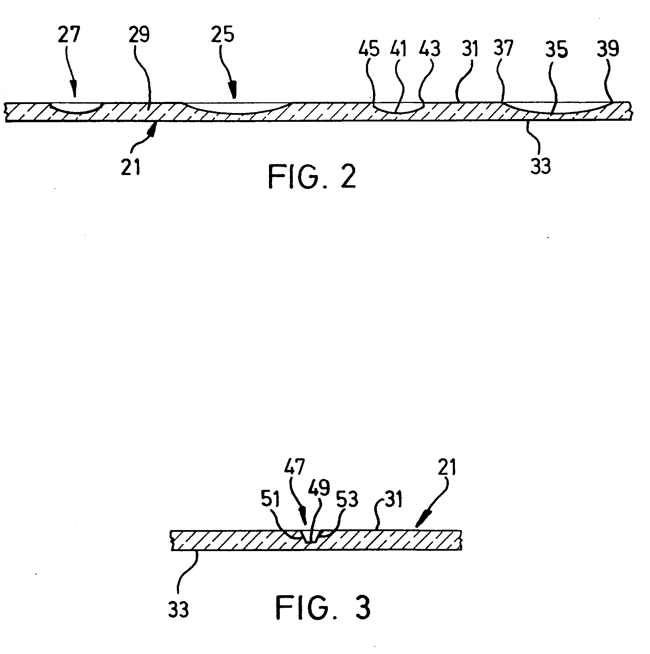

Figure 2 shows the packaging material 21 in longitudinal section through the line of weakness 23. The packaging material 21 has a top side 31 and an underside 33. The long weak points 25 have a longitudinal profile 35 in the form of an arc of a circle which forms the bottom of the weak points 25. The two ends 37, 39 of this longitudinal profile 35 respectively merge gradually at an acute angle into the plane of the top side 31 of the packaging material 21. The bottom of the weak point 25 has the same width over the entire length. If appropriate, however, here too the width of the bottom of the weak points can vary.

In contrast to the weak point 25, each weak point 27 has a longitudinal profile 41 which begins and ends at each of the two ends with a shallow, approximately vertical indentation 43, 45. Only at the lower end of these indentations 43, 45 does the longitudinal profile 41 merge into the shape of an arc of a circle. If the weak points 25, 27 are produced by embossing the packaging material 21, when oppositely directed forces are exerted on the packaging material 21 present on both sides of the line of weakness 23 the structure of the packaging material 21 in the region of the indentations 43, 45 of the short weak points 27 is destroyed earlier than in the region of the long weak points 25. Because of the smaller length of the weak points 27, the destruction forces also take effect sooner at the locations of the indentations 43, 45 than in the region of the long weak points 25. The depth of the weak points 25, 27 is essentially the same and, for example, can amount to 2/3 of the thickness of the packaging material 21.

If appropriate, however, the depth of the weak points 25, 27 can also vary if an even greater differentiation of the material strength of the packaging material is desired at the weak points, in order to increase the freedom from noise even further during the destruction of the line of weakness. This version is suitable for packaging material made of plastic foil or cellophane.

Figure 3 shows a transverse profile 47 of a long weak point 25. The transverse profile 47 corresponds essentially to a U-shape groove and has a bottom 49 which is formed by the longitudinal profile 35 in Figure 2. Side walls 51, 53 form an acute angle which opens towards the top side 31 of the packaging material 21. The upper longitudinal edges of the groove-shaped transverse profile 47 which are formed by the top side 31 and by the side walls 51, 53 should permit a "round" transition in dependence on the properties of the packaging material 21 used. In contrast to the exemplary embodiment described, if required each weak point can have a material weakening over a differing width. Furthermore, if appropriate, weak points of differing width and depth can also be provided in succession.

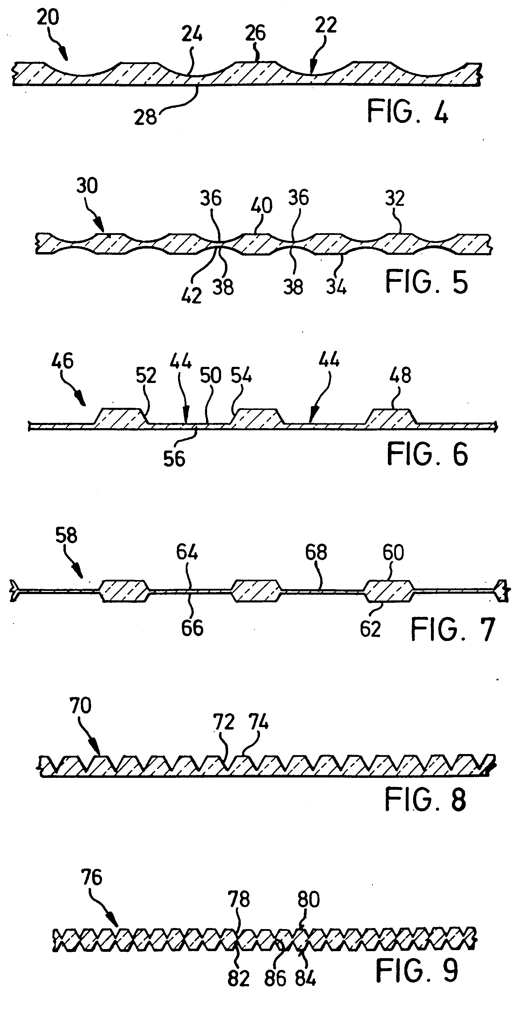

Figures 4 to 11 illustrate further exemplary embodiments of a packaging material having at least one line of weakness consisting of weak points of the packaging material which are arranged at a distance from one another and in succession and which are provided with means for an essentially silent and residue-free destruction of the line of weakness. In these exemplary embodiments, these means likewise consist of weak points of varying material strength of the packaging material.

According to the embodiments of the packaging material in Figures 4 to 9, zones of varying weakness of the packaging material at weak points are provided by a weakening of the structure of the packaging material.

Thus, Figure 4 shows a packaging material 20, the line of weakness of which consists of weak points 22 which have transversely to the direction of the line of weakness a longitudinal profile 24 corresponding to an arc of a circle. In the region of this arc of a circle, the packaging material is weakened first to a slight extent, then to an increasingly greater extent and subsequently once again to an increasingly slighter extent, the transition between these zones of reduced strength being continuous. Located between the weak points 22 is a respective web 26 which, as shown, can be a portion of the packaging material 20, the thickness of which corresponds to that of the remaining packaging material. Figure 4 shows only weak points 22 arranged in succession and separated by the web 26. But it goes without saying that this series of weak points 22 in Figure 4 continues to the left and to the right. The essentially silent destruction of a line of weakness consisting of such weak points 22 provided with an arcuate longitudinal profile can be explained in that, when oppositely directed forces are exerted on the portions of the packaging material 20 which are present on each side of the line of weakness, the destruction of the said packaging material takes place first at locations 28 of heaviest embossing. The unavoidable differences in thickness of the packaging material 20 at the locations 28 of each weak point 22 lead to a destruction of individual weak points 28 which is staggered in time, so that the essentially silent and residue-free destruction of the weak points 22 is obtained.

According to the invention, cellophane having a thickness of more than 10 µm, especially of 20 µm, is preferred at the present time as packaging material. Despite the small thickness of the material, it has been shown, surprisingly, that even within the framework of industrial production it is possible to make weak points in cellophane of this small thickness with the necessary reliability, without the risk that the cellophane will be cut through locally. This is naturally of particular importance for the packaging of tampons for feminine hygiene, which have to be packaged safely against external influences and in a hygienically satisfactory manner, but nevertheless have a packaging capable of being destroyed easily and in as silent and residue-free way as possible. But biologically degradable plastic foils as well as conventional plastic, such as, for example, polypropylene, polyethylene, highly compressed polyethylene and a laminate consisting of plastic and paper can also be used for packs of this type.

Figure 5 shows a modified embodiment of a longitudinal profile of a line of weakness in a packaging material 30, in which the packaging material 30 is provided on its top side 32 and on its underside 34 with respective weak points 36 and 38 in the form of an arc of a circle. It is evident that, in this case, the radii of curvature of the weak points 36, 38 in the form of an arc of a circle are made smaller in comparison with those in Figure 4, and that a web 40 extending between the weak points 36, 38 is made, for example, exactly as long as the weak points 36, 38. The weak points 36 on the top side 32 of the packaging material 30 are made to coincide with the weak points 38 provided on the underside 34 of the packaging material 30, so that, once again, a location 42 of least thickness, at which the material is destroyed first, is obtained half-way along these weak points 36, 38. In the present case too, it is preferable to make the longitudinal profile of the web 40 on the top side 32 and underside 34 in the form of an arc of a circle, so that the zones of varying or reduced strength of the packaging material 30 merge continuously into one another.

Figure 6 shows a further embodiment of weak points 44 in the packaging material 46, in the top side 48 of which a longitudinal profile of weak points 50 corresponds to a U-profile, the legs 52, 54 of which form an acute angle opening towards the top side 48 and the profile web 56 of which is made relatively long in comparison with the height of the legs 52, 54. It is clear that, in the region of the profile webs 56, the packaging material 46 has a greatly reduced thickness which corresponds approximately to one third of the normal thickness of the material.

A profiling, similar to that of Figure 6, of a packaging material 58 can be seen in Figure 7, but here, in a similar way to Figure 5, the packaging material 58 is provided on its top side 60 with weak points 64 and on its underside 62 with weak points 66 which lie congruently one above the other and which have half-way along the height of the thickness of the packaging material 58 common profile webs 68, the thickness of which corresponds only to approximately one fifth of the thickness of the packaging material.

Figure 8 shows a further embodiment of a packaging material 70 which is provided successively in the longitudinal direction of a line of weakness with weak points 72 of triangular longitudinal profile which are separated by profile webs 74 of equal length and of a thickness corresponding to the thickness of the packaging material 70 outside the weak points 72. In this version too, the depth of the weak points 72 varies, so that there is no possibility that the packaging material 70 will break open simultaneously with a cracking noise in the region of least thickness of the latter at the weak points 72.

A further packaging material 76 having weak points 78 on a top side 80 and weak points 82 on an underside 84 of the packaging material 76 can be seen in Figure 9. Once again, the weak points 78, 82 are arranged coincidentally one above the other, thereby forming half-way along the height of the thickness of the packaging material 76 profile webs 86, the thickness of which differs sufficiently to prevent a pack produced from this packaging material 76 from being opened simultaneously with a cracking noise.

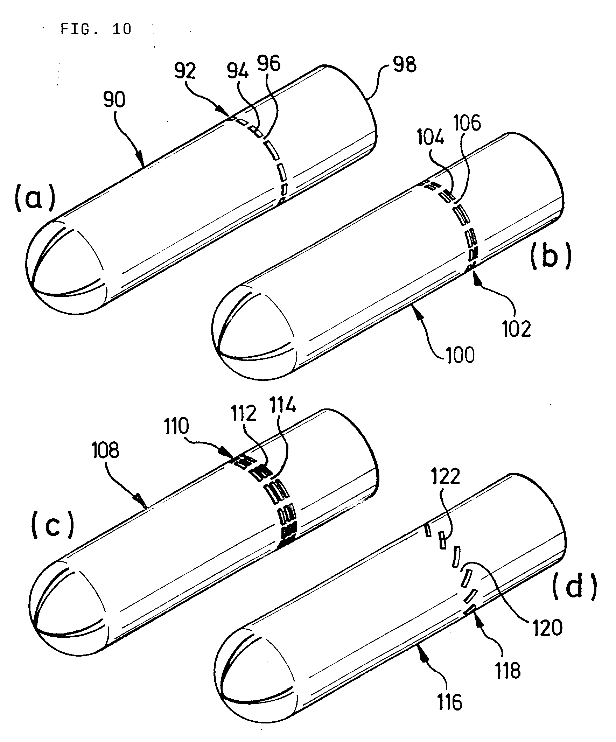

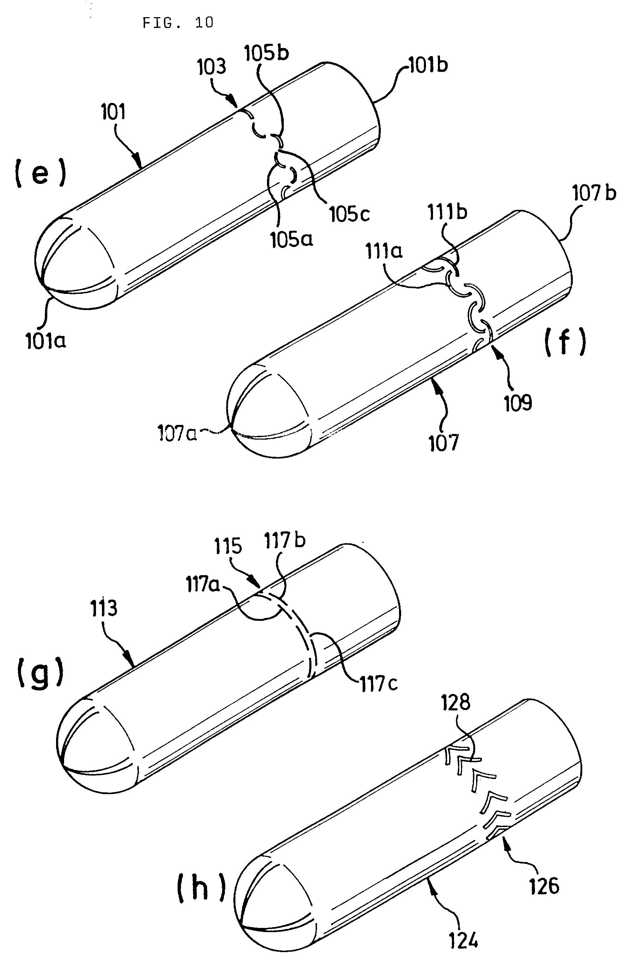

Freedom from noise and freedom from residues of packaging material are especially desirable if the packs are intended for articles of hygiene, especially tampons for feminine hygiene. The discreet handling of packs of this type is an essential psychological precondition for the acceptance of individual tampon packs. Figures 10(a) to 10(e) therefore illustrate exemplary embodiments of individual packs of tampons of this type, which make use of packaging material according to the invention having at least one line of weakness, at the weak points of which the packaging material has a varying weakness.

Thus, Figure 10(a) shows an individual pack 90 having a line of weakness 92 consisting of individual linear weak points 94 which are respectively separated from one another by webs 96. The weak points 94 can be recessed arcuately or in a U-shaped manner in a longitudinal direction of the line of weakness 92, as shown in Figures 1 to 9. The line of weakness 92 is provided at one third of the length of the individual pack 90 from the rear end 98 of the latter.

Figure 10(b) shows an individual pack 100, the line of weakness 102 of which differs from the line of weakness 92 in Figure 10(a) only in that pairs of linear weak points 104 of respectively identical length, located parallel and next to one another and forming a pattern, are arranged around the circumference of the individual tampon pack 100. Webs 106 respectively separate the pairs of weak points 104 in the longitudinal direction of the line of weakness 102.

Figure 10(c) provides a further embodiment of an individual pack 108 for a tampon, having a line of weakness 110 which consists of three respective linear or strip-shaped weak points 112 of equal length which are located parallel and next to one another and which are separated from one another by webs 114 and form a weakening pattern.

Figure 10(d) illustrates an individual pack 116, in which a line of weakness 118 consists of weak points 122 which are separated by webs 120 and which once again, as in the preceding exemplary embodiments, are arranged in a cross-sectional plane of the individual pack 116, but are respectively designed according to a helical pattern. At the same time, the weak points 122 can, if appropriate, overlap with their adjacent ends in the circumferential direction of the line of weakness 118. This design of the weakening patterns of the line of weakness 118 is conducive to the rapid and essentially silent and residue-free destruction of the line of weakness 118.

Figure 10(e) shows an exemplary embodiment of an individual pack 101 for a tampon, the line of weakness 103 of which forms a pattern consisting of successive arcuately curved weak points 105a, 105b. The weak points 105a form arcs which extend convexly in the direction of the front end 101a of the individual pack 101, whilst the weak points 105b are respectively curved convexly towards the rear end 101b of the individual pack 101. In the direction of the line of weakness 103, the arcuate weak points 105a, 105b are separated from one another at relatively small intervals by webs 105c. The arcuate weak points 105a, 105b give the user a "softer" feeling when the line of weakness 103 is being destroyed.

This feeling can also be reinforced by a further embodiment of a line of weakness 109 in Figure 10(f). This shows an individual pack 107 for a tampon, the line of weakness 109 of which consists of weak points 111a, 111b. The weak points 111a are once again curved convexly in the direction of the front end 107a of the individual pack 107, whilst the weak points 111b are also again shaped convexly towards the rear end 107b of the individual pack 107. The arcuately curved weak points 111a, 111b overlap with their ends in such a way that the ends of weak points located in front of and behind an arcuate weak point in the circumferential direction of the individual pack reach into the convex curvature of the weak point. The weak points designed according to Figures 10(e) and 10(f) allow an appreciably softer tearing of the lines of weakness 103 and 109.

Figure 10(g) shows an individual pack 113 for a tampon, provided with a line of weakness 115 which, as in all the embodiments of Figure 10, likewise extends around the circumference of the individual pack. In this embodiment, the line of weakness 115 consists of linear weak points 117a, 117b which are offset relative to one another in the circumferential direction of the individual pack 113 in such a way that the ends of linear weak points 117a, 117b located behind one another mutually overlap, as indicated by the reference symbol 117c. This arrangement of the weak points 117a, 117b also allows a softer tearing of the line of weakness 115 formed by them.

Finally, a very soft tearing effect can also be achieved with an individual pack 124 for a tampon in Figure 10(h), the line of weakness 126 of which consists of weak points 128 which form a pattern and which are V-shaped in the direction of the line of weakness 126. In the present instance, there can be an overlap of the angle vertex of a V-shaped weak point 128 with the free ends of the following V-shaped weak point 128.

Of course, as already indicated, irrespective of the specific design of the above-described weak points, the mutual spacing of the weak points can be varied if an even more silent destruction of the line of weakness or a greater or lesser bending strength of a line of weakness is desired. This variation of the webs connecting the weak points to one another can consist of a continuously increasing or decreasing variation of the web size between the successive weak points. Furthermore, it is also possible, of course, to arrange weak points of differing design or in a differing arrangement of patterns in succession or at specific mutual spacings provided. Thus, it is perfectly possible to provide weak points having one or more of the profiles shown in Figures 1 to 9 jointly for a line of weakness of a pack. It is essential, according to the present invention, that the structure of the packaging material in the region of embossed weak points or the size of the webs forming the weak points between the perforations be varied in such a way that a destruction of the individual weak points which is staggered in time is obtained, so that each individual pack can be opened discreetly, that is to say largely silently, without remains of packaging material being left behind on the packaged tampon.

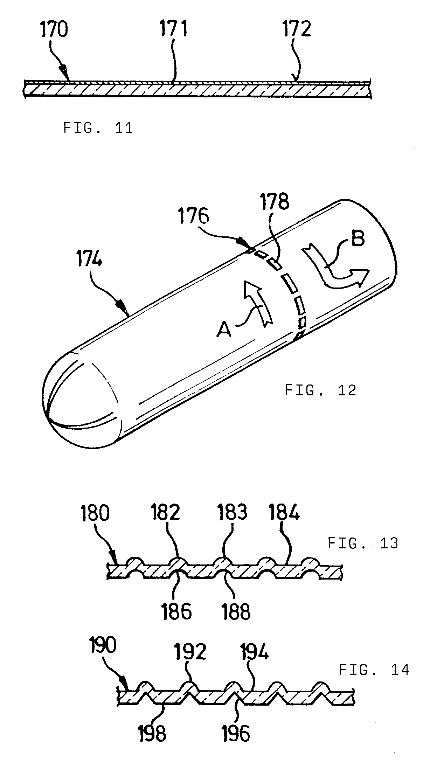

The slip resistance of the surface of the packaging material is an essential precondition for the destruction of the line of weakness of a pack. To satisfy this requirement, Figure 11 shows a longitudinal section through a packaging material 170 made of cellophane, the outside 171 of which is covered with a layer 172 of roughness lacquer. If appropriate, the underside of the packaging material 170 can be provided with an airtight coating.

The roughness lacquer can consist of a clear lacquer, the rough structure of which is brought about by normal, colourless particles. A further possibility for producing the rough structure of the surface of the cellophane can be afforded by the shrink-like drying on of the roughness lacquer as a reaction in the drying stage of a printing machine. Furthermore, the rough structure of the cellophane surface can be brought about by needle-shaped crystals as a reaction in the drying process of the roughness lacquer. When particles are added to the lacquer mass to achieve a greater roughness, these particles can consist of plastic. The basic finishing of the cellophane includes applying a heat-sealing layer to the two sides of the latter, so that the material can be heat-sealed with the front layer against the rear layer in the packaging station. Instead of a normal sealing lacquer, it is preferable to provide the side of the cellophane forming the outside of the pack with a sealing roughness lacquer instead of a normal sealing lacquer, in order thereby to save an additional operation to apply the roughness lacquer to the layer of sealing lacquer.

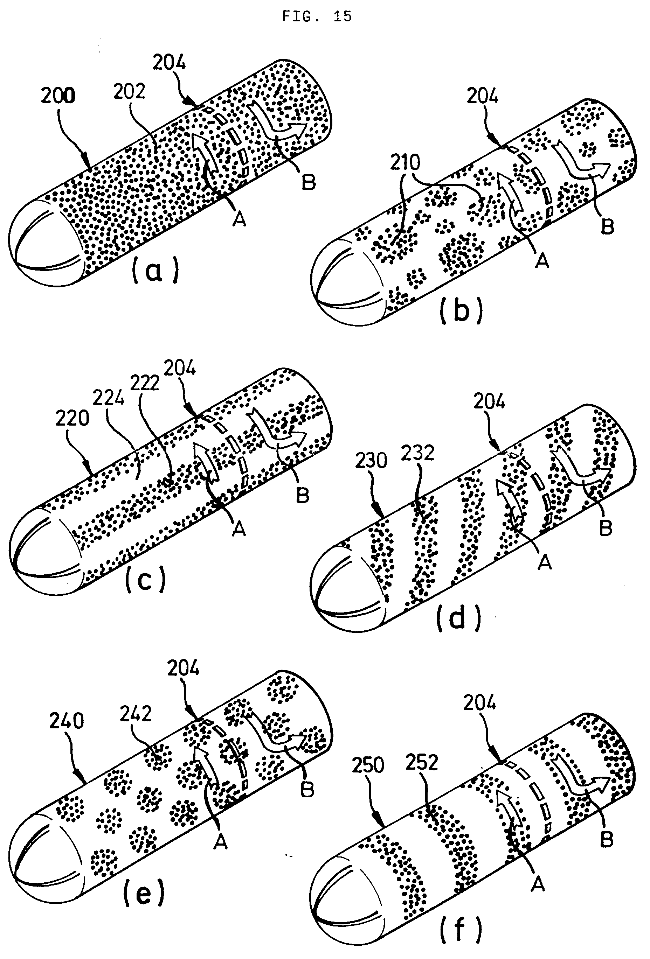

Figure 12 illustrates an individual pack 174 of a tampon for feminine hygiene, having a line of weakness 176 consisting of individual weak points 178, made from the packaging material 170 with the layer of roughness lacquer 172 according to Figure 14. Arrows A and B on both sides of the line of weakness 176 indicate the oppositely directed forces which are exerted by the user when the line of weakness 176 is being destroyed. The part of the pack 174 assigned to the arrow B is pulled off from the rear end of the tampon (not shown), so that a recovery tape fastened in the tampon and bearing against the rear end of the tampon can be detached and extended away from the tampon. The tampon is then grasped at the rear end and the front part of the pack 174 assigned to the arrow A is removed. By inserting a finger into the tampon end, the tampon can thereafter be introduced into the body cavity. This above-described handling of the individual tampon pack 174 applies to all individual tampon packs described herein. Of course, this handling of the pack to open it can also be used for packaged articles of another kind, as long as an oppositely directed rotation of the pack parts located on both sides of the line of weakness is possible in order to destroy the line of weakness guided around the packaged article.

In addition to or instead of the outer layer, consisting of a roughness lacquer, for improving the grip of the packaging material, according to Figures 13 and 14 a packaging material can be provided at least partially with mechanical embossings. Thus, Figure 13 shows a packaging material 180 which has knob-shaped elevations 182. The embossing projects on an outer face 184 of the packing material 180 in the form of round knob heads 183, to which round depressions 188 correspond on an inner face 186.

Figure 14 illustrates a further embodiment of a packaging material 190, in which likewise round knob-shaped elevations 192 project from an outer face 194 of the packaging material 190, in the present case, however, the said elevations being produced by funnel-shaped depressions 196 on an inner face 198 of the packaging material 190.

Figures 15(a) to 15(f) show individual packs according to the invention consisting of the packaging material having various roughening patterns which are each provided with an identical line of weakness 204. An individual pack 200 for a tampon for feminine hygiene is illustrated in Figure 15(a) and is provided completely with mechanical embossings 202 of the type shown in Figure 13 or 14. Arrows A and B in the said Figure indicate once again, as in Figure 12, the direction of the opening forces, by means of which a line of weakness 204 can be destroyed in an essentially silent and residue-free manner. It is understandable that this surface roughness of the individual pack 200 achieves an outstanding grip which allows the desired immediate essentially silent and therefore discreet opening of the individual pack for the tampon.

Whereas Figure 15(b) shows mechanical embossings of the type illustrated in Figure 13 or 14, in the form of spot patterns 210, between which the packaging material is more or less free of mechanical embossings, in Figure 15(c) there are provided embossings 222 extending in the longitudinal direction of an individual pack 220, which extend in a strip-shaped manner in the longitudinal direction of the individual pack 220 and which are separated by parallel strips 224 of packaging material, on which no mechanical embossings are provided.

Figure 15(d) shows an individual pack 230 for tampons for feminine hygiene, in which mechanical embossings 232, which extend helically at a distance from and parallel to one another and which are separated by intermediate strips without embossings, are provided.

In a further individual pack 240 for tampons for feminine hygiene, according to Figure 15(e) clearly mutually spaced spots 242 of mechanical embossings are distributed over the surface of the individual pack 240 at essentially identical mutual spacings.

Finally, Figure 15(f) shows an individual pack 250 for tampons for feminine hygiene, in which annular strips 252 of mechanical embossings, which are arranged at a mutual spacing in the longitudinal direction of the individual pack 250, are provided. Depending on the type and thickness of the packaging material and on the design of the line of weakness 204, it can therefore be expedient to provide the packaging material completely with mechanical embossings according to Figure 15(a) or else to equip only part regions of the latter with mechanical embossings of this type, as shown in Figures 15(b) to 15(f).

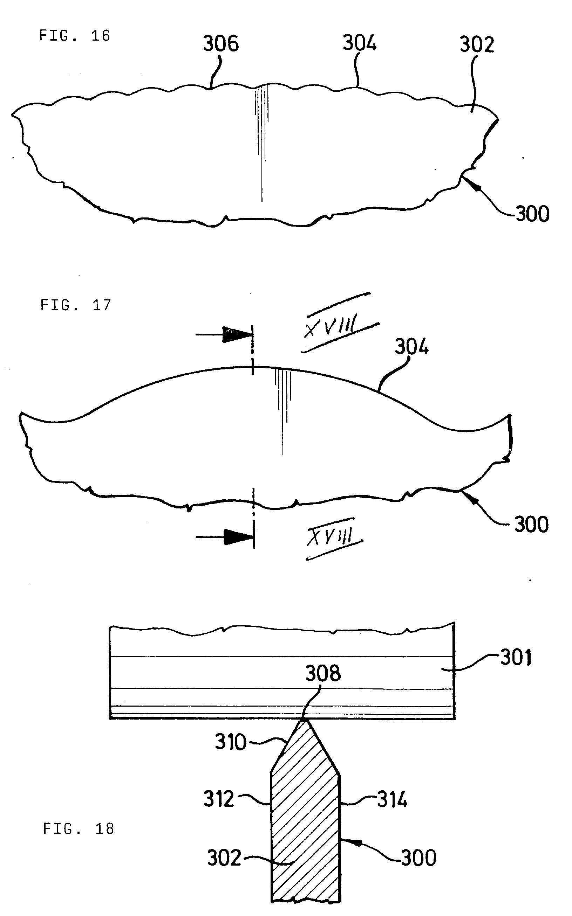

Figures 16 to 18 show an apparatus for making weak points in a packaging material, the apparatus consisting of a circular, freely rotatable weakening disc 300 having weakening teeth 302 on its outer circumference. In the present instance, weakening teeth 302 are equipped with weakening knife edges 304 which, as shown in Figures 16 and 17, are curved outwards in the form of an arc of a circle and are distributed at a pitch of 2° over the circumference of the weakening disc 300. The radius of curvature of the weakening knife edges 304 curved radially outwards amounts, for example, to 5 mm. It is clear that the weakening knife edges 304 of adjacent weakening teeth 302 merge continuously into one another, interspaces 306 being curved radially inwards in the form of an arc of a circle in relation to the axis of the weakening disc 300, of which interspaces 306 the radius of curvature is naturally calculated as substantially smaller than that of the weakening knife edges and can amount, for example, to 0.2 mm.

It is evident from Figure 18 that an outer weakening face 308 of the weakening teeth 302 extends parallel to the axis of the weakening disc 300, so that, as indicated, for example, in Figures 1 and 3, a plane or curved bottom at the weak points is produced in the region of the weak points of the packaging material. A transverse profile 310 of each weakening tooth 302 widens from the outer longitudinal edges of this weakening face 308 as far as the parallel outer faces 312, 314 of the weakening disc 300. There thus takes place in conjunction with the convexly curved weakening knife edges 304 of the weakening teeth 302 a gentle compression of the structure of the packaging material, during which, as tests have shown, a cutting through of the packaging material is reliably prevented in spite of the small thickness of at least 10 µm of the latter.

An essential contribution to this is made by the smooth transition which is formed in the individual weakening knife edges 304 of adjacent weakening teeth 302 by the interspaces 306 concavely curved in the form of an arc of a circle in relation to the axis of the weakening disc 300.

The weakening disc 300 according to Figures 16 to 18, like the weakening discs described further below, is employed in conjunction with a smooth-cylindrical counterroller 301 which is designed as a free-wheel roller. The smooth surface of the counterroller 301 preferably consists of a hard material, such as, for example, steel, so that the desired weakening is achieved as a result of a partial destruction of the structure of the cellophane. However, under some circumstances, depending on the type of packaging material used, for example a plastic foil, a counterroller 301 having a less hard surface consisting, for example, of a metal alloy or of a plastic coating can also come under consideration.

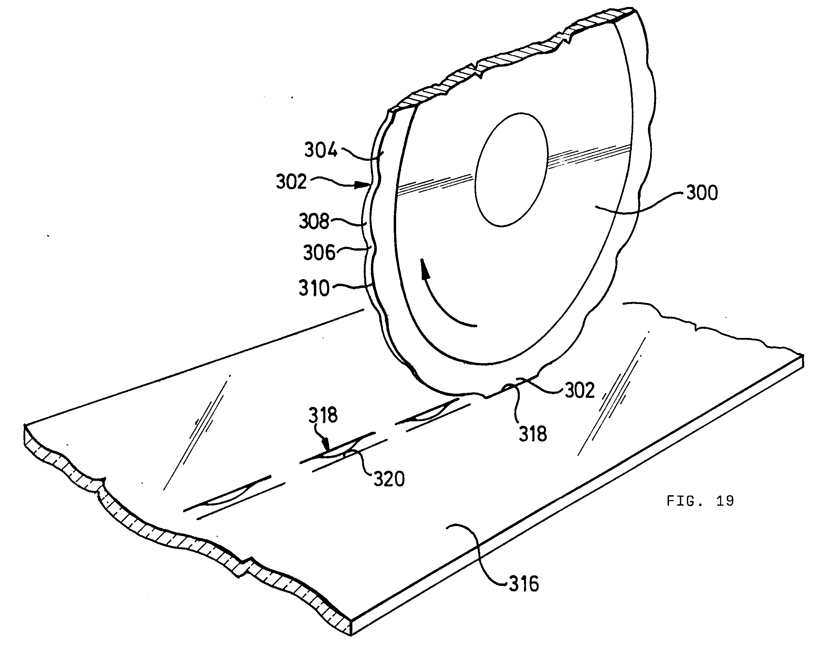

An exemplary embodiment of a process for making a line of weakness in a cellophane web 316 by means of the weakening disc 300 in Figures 16 to 18 is described below by reference to Figure 19, the counterroller 301 not being shown. In Figure 19, one of the weakening teeth 302 has penetrated completely into the cellophane web 316 and embosses a weak point 318 in the same way as was previously carried out for the preceding weak points 318 of the cellophane 316 by corresponding weakening teeth 302. Because the weakening teeth 302 are in the form of an arc of a circle in the circumferential direction of the weakening disc 300, each weakening tooth 302 penetrates smoothly into the cellophane web 316, the cellophane being compressed by approximately one third of its thickness by the outer weakening face 308 of each weakening tooth 302, but not being cut through. At the same time, as mentioned, the structure of the cellophane, known to be relatively brittle, is partially destroyed, without the cellophane thereby losing its tightness against the entry of air or foreign bodies. With a width of each weakening tooth 302 of 0.2 to 0.5 mm, preferably 0.35 mm, and with a diameter of at least 80 mm of the weakening disc 300 and a diameter of the counterroller 301 smaller than this, satisfactory embossing results were achieved when an embossing pressure of 2 to 5 bar was exerted.

For a weak-point embossing of the packaging material on one side, the web of packaging material is drawn through the gap between the weakening disc and the counterroller, the weakening disc and the counterroller being set in rotation. If the packaging material is to be provided with weak-point embossings on both sides, as is shown, for example, in Figures 5, 7 and 9 described further above, however, it is recommended to drive the weakening disc and the counterroller in synchronism.

The weak-point embossings 318 in the top side of the cellophane web 316 each have a weak-point bottom 320 which, as a result of the differing exertion of pressure of the weakening teeth 304 in the form of an arc of a circle, is slightly inclined at the front end, this corresponding to a slight weakening and destruction of the cellophane structure. As is evident, a smooth transition occurs between the initially light embossing and the heaviest or deepest embossing half-way along each weak point 318; on the second rear length portion of the weak point 318, the embossing extends exactly in reverse, namely from the heaviest embossing in the longitudinal centre of the weak point 318 with an ever lower embossing pressure as far as the rear end of the weak point 318, where the embossing merges smoothly into the unembossed surface of the cellophane web 316. Depending on the type of cellophane or another packaging material, especially its thickness, if appropriate a second weakening disc 300 identical to the first weakening disc 300 can be provided as a counterroller, in order to provide the packaging material with a corresponding weak-point embossing also on the underside. In this case, a synchronous rotational drive of the two weakening discs is recommended. At all events, for forming the weak-point embossing according to the invention in cellophane or in other packaging materials having similar properties, it is important that the weakening teeth 302 should penetrate into the packaging material smoothly or gradually, at least shortly after the start of embossing, in order to prevent a cutting through of the packaging material which, in the event of destruction, could under some circumstances lead to a crumbling away of very fine particles of material.

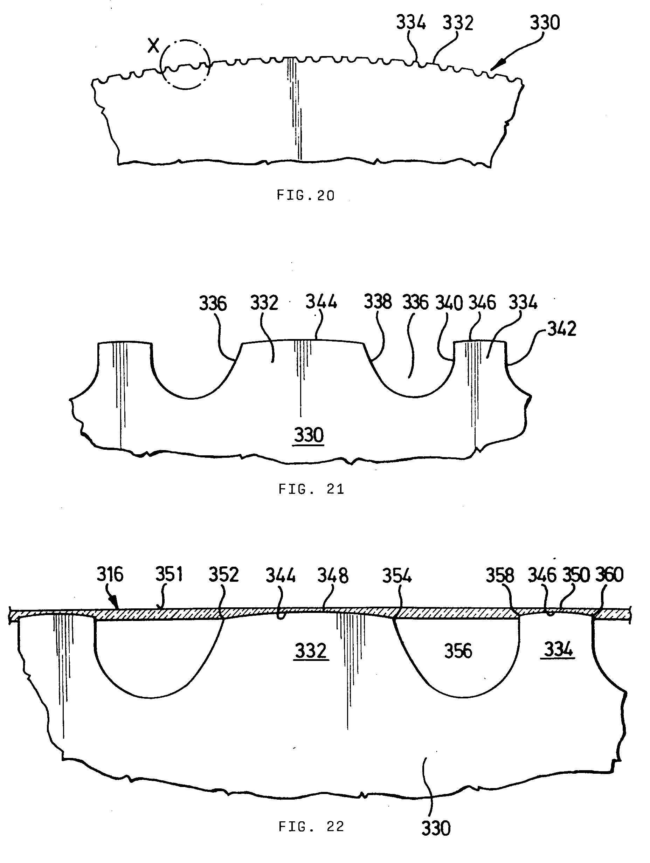

An embodiment of an apparatus for making lines of weakness 23 of the especially advantageous embodiment shown in Figures 1 to 3 is illustrated in Figures 20 to 22. In a weakening disc 330 of this apparatus, long weakening teeth 332 and short weakening teeth 334 are alternately arranged in succession over its circumferential length. The spacing of the long weakening teeth 332 and the short weakening teeth 334 is made the same in each case and, in the present exemplary embodiment, amounts, for example, to 1°. It follows from this that the spacing of two long weakening teeth 334 and of two short weakening teeth 332 amounts to 2° in each case. In the present embodiment, the long weakening teeth 334 are 0.45 mm long and the short weakening teeth 334 0.2 mm long. As shown especially in Figures 21 and 22, both the long weakening teeth 332 and the short weakening teeth 334 are curved radially outwards. The radius of curvature of the long weakening teeth 334 amounts to 2.0 mm and that of the short weakening teeth 334 to 0.7 mm. It is evident, furthermore, that front and rear tooth flanks 336, 338 of the long weakening teeth 332 are curved to a lesser extent than circumferentially front and rear tooth flanks 340, 342 of the short weakening teeth 334, so that the tooth flanks 336, 338 of the long weakening teeth 332 respectively form an obtuse angle with their weakening knife edge 344. In contrast to this, the tooth flanks 340, 342 form a right angle with the weakening knife edge 346 of the short weakening teeth 334. In the present exemplary embodiment, the radius of curvature of the front and rear tooth flanks 336, 338 of the long weakening teeth 332 amounts to 0.465 mm and the radius of curvature in the region of the front and rear tooth flanks 340, 342 of the short weakening teeth 334 to 0.15 mm. The transverse profile of the long weakening teeth 332 and short weakening teeth 334 can correspond to that in Figure 18. At the same time, the outer or active tip face or weakening face of each weakening tooth 332, 334 can have a width of 0.35 mm in accordance with the weakening face 308 in Figure 18.

Figure 22 clearly reveals that the long weakening tooth 332 and the two short weakening teeth 334 penetrate only gradually into the cellophane web 316 with the radius of their weakening knife edges 344 and 346 respectively and reemerge from the cellophane web 316 just as gradually. As a result of the differing length of the weakening knife edges 344, 346 of the weakening teeth 332, 334, correspondingly long or short weak points 348, 350 are obtained, and as Figure 22 clearly shows, the long and short weakening teeth 332, 334 more or less destroy the structure of the cellophane 316 to a depth of approximately 2/3 of the thickness of the cellophane 316, whilst the structure on the side 351 of the cellophane web 316 facing away from the weakening disc 330 is preserved completely and therefore allows a sealing packaging of an article with the cellophane.

It is critical for the described effect of this weakening disc 330 that the weak points 348, 350 which are embossed by the long weakening teeth 332 and the short weakening teeth 334 are different. This is based on the fact that a front tooth edge 352 and a rear tooth edge 354 of each of the long weakening teeth 332 are located, in its embossing position completely penetrated into the cellophane 316, approximately exactly in the plane of the surface 356 of the cellophane web 316 which confronts the weakening disc 330. In contrast, a front tooth edge 358 and a rear tooth edge 360 of each short weakening tooth 334 are so arranged that, in the completely penetrated position of the short weakening tooth 334 shown in Figure 22, they are located within the cellophane 316 and form the indentations designated by 41 and 43 in Figure 2. As Figure 22 shows, the tooth edges 358, 360 have penetrated into the cellophane 316 to approximately one third of the thickness of the latter. This differing embossing of the cellophane web 316 by the front and rear tooth edges 352, 354 of the respective long weakening teeth 332 and of the front and rear tooth edges 358, 360 of the respective short weakening teeth 334 produces weak points 348 and 350 which have, at the four points of penetration of the said tooth edges, fine material webs, at which, during the destruction of the line of weakness formed by the embossing points, the cellophane 316 bursts apart in a manner staggered in time and therefore somewhat silently. As a result of the differing length of the weakening teeth 332, 334, the spacings between them can be kept the same, as mentioned. If the cellophane 316 is exposed to oppositely directed forces on both sides of the line of weakness in order to destroy the line of weakness, because of the arrangement of the short weakening teeth 334 the material between the long weakening teeth 332 is not stretched in the direction of the line of weakness, but bursts apart, because the short weakening teeth 334 penetrate with their tooth edges 358, 360 into the cellophane 316 and produce a weak point at which the material tears. This prevents any fraying or peeling off of small residues of the cellophane 316.

Tests with a cellophane pack for tampons for feminine hygiene were conducted, use being made of cellophane with a line of weakness which was made by the freely rotatable weakening disc 330 according to Figures 20 to 22. With the use of weakening teeth 332, 334 with a width of the outer weakening face of 0.35 mm, the best result was obtained at a pneumatic contact pressure of 4.0 bar of the weakening disc 330 against the cellophane and a likewise freely rotatably mounted counterroller made of steel. The cellophane web was drawn at a speed of 150 m/min from a stock roll (not shown) through the gap between the weakening disc and the counterroller. The thickness of the cellophane amounted to 22/100 mm, there remaining after the embossing, at the embossed weak points, a material thickness of 7/1000 mm guaranteeing the sealing of the pack.

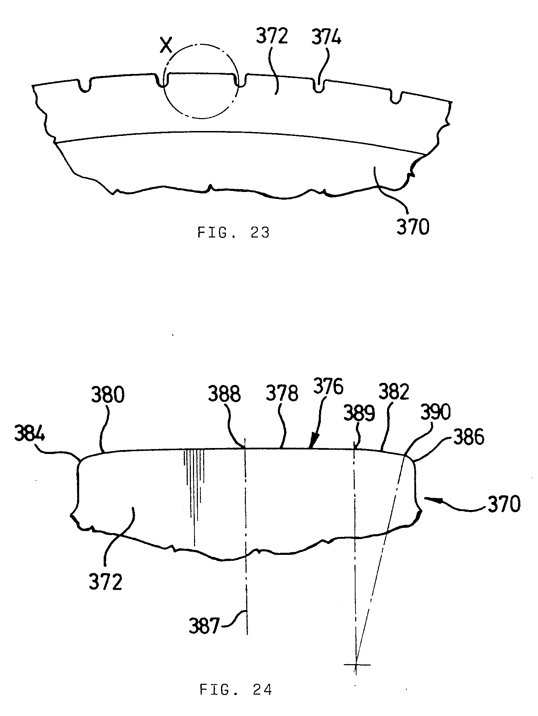

Figures 23 and 24 show a further embodiment of a weakening disc 370 with weakening teeth 372 of respectively identical size and dimensions, between which respective identical interspaces 374 are arranged. The weakening disc 370 has, again, a diameter of at least 80 mm. In this case, each weakening tooth 372 extends over an arc length of 4°, the interspaces 374 having a length of 0.35 mm in the circumferential direction. The weakening teeth 372 have weakening knife edges 376 which are convexly curved radially outwards. Each weakening knife edge 376 is composed of three curved portions of differing size, namely a middle main portion 378 as well as a front and rear end portion 380, 382 and a front and rear corner portion 384, 386. The middle main portion 378 of the weakening knife edge 378 is an arc of a circle having a radius of curvature of 1.5 mm. The middle main portion 378 extends on both sides of an end point 388 of an axis of symmetry 387 of the weakening knife edge 372 over a total length of 1.5 mm to more sharply curved end portions 380, 382 which each have a radius of curvature of 1.5 mm. The portion 382 extends from a point 389 to a point 390 which separates the rear end portion 382 from the rear corner portion 386, the convex curvature of which has a radius of 0.1 mm. The other end of the weakening knife edge 372 is correspondingly shaped. The transverse profile of the weakening teeth 372 again corresponds to that in Figure 18, and only the outer weakening face of the weakening teeth 372 can vary between a width of 0.25 and 0.5 mm. The embossing effect of the weakening teeth 372 in Figures 20 and 21 is similar to that of the long weakening teeth 332 in Figures 17 to 19, but is not identical because, in the present exemplary embodiment, the corner portions 384, 386 are fully rounded and therefore each of the weakening teeth 372 gradually penetrates smoothly into the packaging material, such as cellophane, and is also moved out of the packaging material again likewise gradually with the other end of the weakening tooth 372.

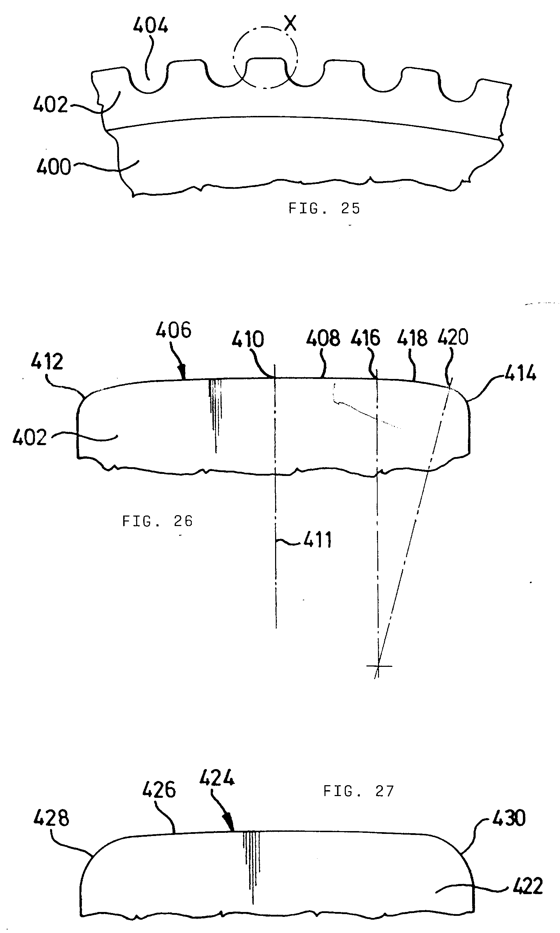

A further embodiment of a weakening disc 400 is illustrated in Figures 25, 26 and 27. This weakening disc 400 has weakening teeth 402 having a division of an arc length of 4°. The weakening teeth 402 are separated from one another by webs or interspaces 404 which, as seen in the circumferential direction, are made approximately exactly as long as the weakening teeth 402. In the present exemplary embodiment, this clear width between successive weakening teeth 402 amounts to 1.15 mm. It emerges from Figure 26 that a weakening knife edge 406 of the weakening tooth 402 has a middle main portion 408 which is curved in the form of an arc of a circle and which extends from an end point 410 of an axis of symmetry 411 of the weakening knife edge 406 to a front corner portion 412 and a rear corner portion 414 over a length of 0.35 mm respectively. A further curved portion extends respectively forwards and rearwards from a front end (not designated) and a rear end 416 of this middle main portion 408, and as before only the rear curve portion 418 is designated, this extending as far as a point 420, at which the rear, rounded corner portion 414, corresponding to the front corner portion 412 commences. The front and rear corner portions 412, 414, each have a radius of curvature of 0.1 mm.

Figure 27 illustrates a modified embodiment of a weakening tooth 422 of the weakening disc 400, of which weakening tooth the weakening knife edge 424 has a middle, slightly convexly curved length portion 426, followed at the front and rear end only by rounded corner portions 428, 430 which each have a radius of curvature of 0.2 mm.

A particular feature of the weakening disc 400 is that its outer contour together with the weakening teeth 402 and 422 and the interspaces or webs 404 is completely wire-eroded. Consequently, the weakening face of the weakening knife edges 406 has some roughness which allows a better frictional connection of the cellophane between the weakening disc 400 and a smooth counterroller (not shown) and therefore a highly accurate embossing of the cellophane web.

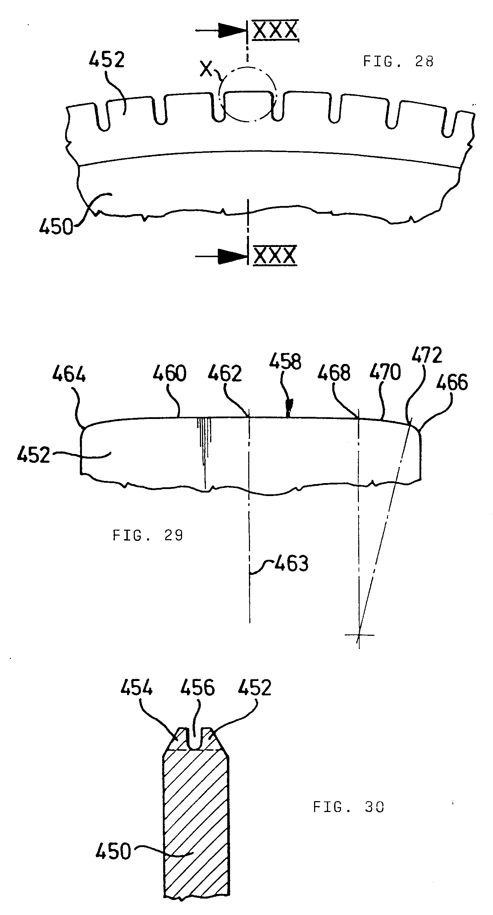

Figures 28, 29 and 30 illustrate a further version of a weakening disc 450 having twin weakening teeth 452, 454 which are arranged at a small mutual spacing of 0.35 mm and which each extend over an arc length of 3°. The twin weakening teeth 452, 454 each have a height of 0.5 mm and, as shown in Figure 28, are made of equal length in the circumferential direction of the weakening disc 450 and, according to Figure 30, are arranged parallel to one another and are separated from one another by a middle circumferential groove 456. The middle circumferential groove 456 has a width of 0.5 mm, whilst the outer weakening face of the twin weakening teeth 452, 454 amounts respectively to 0.3 mm. As in all other instances, the outsides of the twin weakening teeth 452, 454 form a tapering angle with the outer edge of the weakening disc 450 which can amount, for example, to 60°.

According to Figure 29, each twin weakening tooth 452, 454 is equipped with a weakening knife edge 458 composed of a radially outwardly curved middle main portion 460 which extends from an end point 462 of an axis of symmetry 463 of each of the weakening teeth 452, 454 to a front corner portion 464 and to a rear corner portion 466 over a length of 0.5 mm. Here too, the front end (not shown) of the main portion 460 and its rear end 468 are followed by end portions, of which only the rear one is designated by 470. These end portions at the front and rear end of the twin weakening teeth 452, 454 have a radius of curvature of 1.5 mm. One end 472 of the rear end portion 470 is followed by the rear corner portion 466 which has a radius of curvature of 0.1 mm. The front corner portion 464 is correspondingly rounded. A weak point produced by the twin weakening teeth 452, 454 has a particular effect in the destruction of the line of weakness formed by this weak point in the cellophane or in a similar packaging material. In particular, since an unembossed middle web formed by the middle circumferential groove 456 remains between the twin weakening teeth 452, 454, a reinforced shearing effect is exerted on the weak points before the cellophane bursts in the region of the weak points. The method of embossing is thus akin to that explained by means of the three preceding exemplary embodiments. The twin weakening teeth 452, 454 penetrate smoothly or gradually into the cellophane with their rounded corner portions 464, 466 and are moved out of the cellophane just as smoothly with their rear rounded end edge.

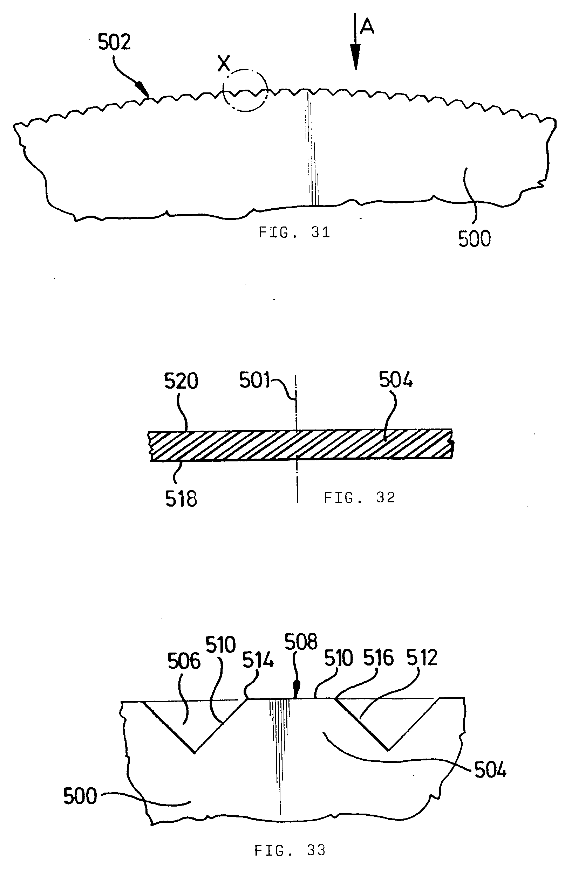

A further embodiment of a weakening disc 500 is illustrated in Figures 31 to 33. This weakening disc 500 is equipped on its outer edge with an inclined toothing 502, individual weakening teeth 504 according to Figure 31 forming an angle of 55° with an axis of rotation 501 of the weakening disc 500. The inclined toothing 502 has a tooth division which respectively corresponds to an arc length of 1°. The length of each inclined weakening tooth 504, as measured over the circumference of the weakening disc 500, amounts to 0.27 mm. The individual inclined weakening teeth 504 are separated from one another by webs or interspaces 506, mutually opposite straight flanks 510, 517 of adjacent weakening teeth 504 forming an angle of 90°, so that, as shown in Figure 33, each weakening tooth 504 has a trapezoidal shape. The front and rear flanks 505, 507 of each inclined weakening tooth 504 form respectively with its plane weakening face 508 an obtuse angle and therefore a relatively pronounced front edge 514 and a correspondingly pronounced rear edge 516. A weak-point embossing formed by these inclined weakening teeth 504 is characterised by a sharper cutting of the cellophane by the front edge 514 and the rear edge 516 and, furthermore, by a relatively strong cutting of the circumferential edges 518, 520 of each weakening tooth 504 which are located on the two main sides of the weakening disc 500.

## Claims

1. Individual, substantially air-tight pack (100) containing a digital tampon for feminine hygiene of which the rear end is provided with a recovery tape, said pack comprising:
packaging material enclosing said individual tampon and having means arranged around the circumference of the individual tampon pack (100) for opening the package,
characterized by
said means for opening the package being provided by at least one line of weakness (23; 92; 102; 110; 118; 126; 132; 146; 148; 162; 176; 204); said at least one line of weakness is a row of weak points (22, 25, 27, 28; 36; 38; 44; 64; 66; 72; 78; 82; 86; 94; 105a, 105b; 111a, 111b; 112; 117a, 117b; 122; 128; 136; 150, 152; 178) consisting of embossments or depressions;
the packaging material having a varying thickness along these weak points, so that said weak points ensure an air-tight, hygienically satisfactory packaging of said tampon until the largely silent and residue-free destruction of the line of weakness for the purpose of opening the pack by acting upon package portions (A, B) being positioned at both sides of the row of weak points.

2. Pack according to claim 1, characterized in that said line of weakness is arranged around the circumference of the individual tampon pack (100) at a distance of approximately one third of the length of the individual pack (100) from that end (98) of the latter which is assigned to said rear tampon end provided with the recovery tape.

3. Pack according to claim 1, characterized in that the weak points (22, 25, 27; 36) consist of arcuate depressions, as seen in mid-longitudinal section.

4. Pack according to claim 1, characterized in that the ends of the weak points (27) are formed by shallow, essentially vertical indentations (43, 45), between which extends a bottom (49) of the weak points (27), said bottom being arcuate in longitudinal profile.

5. Pack according to claim 1, characterized in that the weak points (44; 64, 66) have an essentially U-shaped longitudinal profile.

6. Pack according to claim 1, characterized in that the weak points (72; 78) have a V-shaped longitudinal profile.

7. Pack according to anyone of claims 1 to 6, characterized in that the weak points (36, 38; 64, 66) are provided on the top side (32; 60; 80) and underside (34; 62; 84) of the packaging material (30; 58; 76).

8. Pack according to claim 7, characterized in that the weak points (366, 38; 64, 66; 78, 82) are coincidental on the top side (32, 60; 80) and underside (34; 62; 84).

9. Pack according to claim 6 or 7, characterized in that the weak points (25, 27) which have a differing length in the direction of the line of weakness are arranged alternately in succession.

10. Pack according to claim 1, characterized in that webs (26; 29; 40), each of the same dimension, are arranged between the weak points (22; 25, 27; 28; 36, 38) in and/or transversely to the direction of the line of weakness.

11. Pack according to claim 1, characterized in that the weak points are formed by weakening patterns.

12. Pack according to anyone of claims 1 to 11, characterized in that the outside (184) of the packaging material (180) is provided at least partially with knob-shaped elevations (182; 192) improving the grip.

13. Pack according to anyone of claims 1 to 12, characterized in that the packaging material of the pack is coated.

14. Pack according to claim 13, characterized in that the coating is an outer layer.

15. Pack according to claim 14, caracterized in that the outer layer consists of a roughness lacquer (172).

16. Pack according to anyone of claims 1 to 15, characterized in that the packaging material of the pack consists of cellophane.

17. Pack according to anyone of claims 1 to 15, charactzerized in that the packaging material of the pack consists of plastic.

18. Pack according to claim 17, characterized in that the plastic is biologically degradable.

19. Pack according to anyone of claims 1 to 18, characterized in that the packaging material for the pack is more than 10 microns thick.

20. Pack according to claim 19, characterized in that the thickness of the packaging material amounts to 20 microns.

21. Pack according to claim 1, characterized in that the weak points (94) are recessed arcuately in a longitudinal direction of the line of weakness (92).

22. Pack according to claim 21, characterized in that the weak points (94) are recessed in a U-shaped manner in longitudinal direction of the line of weakness.

23. Pack according to claim 1, characterized in that the line of weakness consists of pairs of linear weak points ((104) of respectively identical length, located parallel and next to one another.

24. Pack according to claim 1, characterized in that the line of weakness (110) consists of three respective strip shaped weak points (112) of equal length which are located parallel and next to one another.

25. Pack according to claim 1, characterized in that the line of weakness (118) consists of weak points (122) which are respectively designed according to a helical pattern.

26. Pack according to claim 25, characterized in that the said weak points (122) overlap with their adjacent ends in the circumferential direction of the line of weakness (118).

27. Pack according to claim 1, characterized in that the line of weakness (103) forms a pattern consisting of successive arcuately curved weak points (105a, 105b; 111a, 111b) forming arcs which extend convexly in the direction of the front end (101a) of the individual pack (101; 107), whilst the weak points (105b) are respectively curved convexly towards the rear end (101b; 107b) of the individual pack (101; 107).

28. Pack according to claim 27, characterized in that the said arcuate weak points (105a, 105b) are separated from one another at relatively small intervals by webs (105c).

29. Pack according to claim 27, characterized in that the arcuately curved weak points (111a, 111b) overlap with their ends in such a way that the ends of weak points located in front of and behind an arcuate weak point in the circumferential direction of the individual pack (107) reach into the convex curvature of the weak point.

30. Pack according to claim 1, characterized in that the line weakness (115) consists of linear weak points (117a, 117b) which are offset relative to one another in the circumferential direction of the individual pack (113) in such a way that the ends of linear weak points (117a, 117b) located behind one another mutually overlap.

31. Pack according to claim 1, characterized in that the line of weakness (126) consists of weak points (128) which form a pattern and which are V-shaped in the direction of the line of weakness (126).

32. Pack according to claim 31, characterized in that there is provided an overlap of the angle vertex of a V-shaped weak point (128) with the free ends of the following V-shaped weak point (128).

33. Pack according to claim 1, characterized in that the outside of the packaging material (170) being made of cellophane is covered with a layer (172) of roughness lacquer.

34. Pack according to claim 1, characterized in that the underside of the packaging material being made of cellophane is provided with an airtight coating.

35. Pack according to claim 33, characterized in that the rough structure of a lacquer is brought about by normal particles.

36. Pack according to claim 33, characterized in that the rough structure of the cellophane surface is afforded by a shrink-like drying of the roughness lacquer as a reaction in the drying stage of a printing machine.

37. Pack according to claim 33, characterized in that the rough structure of the cellophane surface is provided by needle-shaped crystals as a reaction in the drying process of the roughness lacquer.

38. Pack according to claim 33, characterized in that the rough structure of the cellophane surface is achieved by plastic particles added to the lacquer mass.

39. Pack according to claim 33, characterized in that a heat sealing layer is applied to the two sides of the cellophane.

40. Pack according to claim 39, characterized in that the side of the cellophane forming the outside of the pack is provided with a sealing roughness lacquer.

41. Pack according to claim 1, characterized in that the outer face (184; 194) of the packaging material (180; 190) for the pack is provided at least partially with mechanical embossings (182; 183; 192; 202; 222; 232; 242; 252) and corresponding depressions (188; 196) on an inner face (186; 198) of said packaging material.

## Patentansprüche

1. Einzelne, im wesentlichen luftdichte Verpackung (100), die einen Digitaltampon für die Frauenhygiene enthält, dessen hinteres Ende mit einem Rückholband versehen ist, wobei die Verpackung umfaßt:
ein Verpackungsmaterial, das den einzelnen Tampon umschließt und das ein Mittel aufweist, das um den Umfang der einzelnen Tamponverpackung (100) herum zum Öffnen der Verpackung angeordnet ist,
dadurch gekennzeichnet, daß
das Mittel zum Öffnen der Verpackung aus mindestens einer Schwächungslinie (23; 92; 102; 110; 118; 126; 132; 146; 148; 162; 176; 204) besteht; die mindestens eine Schwächungslinie eine Reihe von Schwachstellen (22, 25, 27, 28; 36; 38; 44; 64; 66; 72; 78; 82; 86; 94; 105a, 105b; 111a, 111b; 12; 117a, 117b; 122; 128; 136; 150, 152; 178) ist, die aus Prägungen oder Vertiefungen bestehen;
das Verpackungsmaterial eine sich verändernde Dicke längs dieser Schwachstellen hat, so daß die Schwachstellen eine luftdichte, hygienisch zufriedenstellende Verpackung des Tampons bis zu der weitgehend geräuschfreien und rückstandsfreien Zerstörung der Schwächungslinie zum Zwecke des Öffnens der Verpackung durch Einwirken auf Verpackungsteile (A, B) sicherstellen, die an beiden Seiten der Reihe von Schwachstellen angeordnet sind.

2. Verpackung nach Anspruch 1, dadurch gekennzeichnet, daß die Schwächungslinie um den Umfang der einzelnen Tamponverpackung (100) in einem Abstand von annähernd einem Drittel der Länge der einzelnen Verpackung (100) von demjenigen Ende (98) der letzteren angeordnet ist, das dem hinteren, mit dem Rückholband versehenen Tamponende zugeordnet ist.

3. Verpackung nach Anspruch 1, dadurch gekennzeichnet, daß die Schwachstellen (22, 25, 27; 36), im Mittellängsschnitt gesehen, aus bogenförmigen Vertiefungen bestehen.

4. Verpackung nach Anspruch 1, dadurch gekennzeichnet, daß die Enden der Schwachstellen (27) von schwachen, im wesentlichen senkrechten Einkerbungen (43, 45) gebildet sind, zwischen denen sich ein im Längsprofil bogenförmiger Boden (49) der Schwachstellen (27) erstreckt.

5. Verpackung nach Anspruch 1, dadurch gekennzeichnet, daß die Schwachstellen (44; 64, 66) ein im wesentlichen U-förmiges Längsprofil aufweisen.

6. Verpackung nach Anspruch 1, dadurch gekennzeichnet, daß die Schwachstellen (72; 78) ein V-förmiges Längsprofil aufweisen.

7. Verpackung nach einem der Ansprüche 1 bis 6, dadurch gekennzeichnet, daß die Schwachstellen (36, 38; 64, 66) auf der Oberseite (32; 60; 80) und Unterseite (34; 62; 84) des Verpackungsmaterials (30; 58; 76) vorgesehen sind.

8. Verpackung nach Anspruch 7, dadurch gekennzeichnet, daß die Schwachstellen (36, 38; 64, 66; 78, 82) auf der Oberseite (32, 60; 80) und Unterseite (34; 62; 84) deckungsgleich sind.

9. Verpackung nach Anspruch 6 oder 7, dadurch gekennzeichnet, daß die Schwachstellen (25, 27), die in Richtung der Schwächungslinie eine unterschiedliche Länge aufweisen, abwechselnd hintereinander angeordnet sind.

10. Verpackung nach Anspruch 1, dadurch gekennzeichnet, daß Stege (26; 29; 40) von jeweils gleicher Abmessung zwischen den Schwachstellen (22; 25, 27; 28; 36, 38) in und/oder quer zu der Richtung der Schwächungslinie angeordnet sind.

11. Verpackung nach Anspruch 1, dadurch gekennzeichnet, daß die Schwachstellen von Schwächungsmustern gebildet sind.

12. Verpackung nach einem der Ansprüche 1 bis 11, dadurch gekennzeichnet, daß die Außenseite (184) des Verpackungsmaterials (180) mindestens teilweise mit den Griff verbessernden, nockenförmigen Erhebungen (182; 192) versehen ist.

13. Verpackung nach einem der Ansprüche 1 bis 12, dadurch gekennzeichnet, daß das Verpackungsmaterial der Verpackung beschichtet ist.

14. Verpackung nach Anspruch 13, dadurch gekennzeichnet, daß die Beschichtung eine Außenschicht ist.

15. Verpackung nach Anspruch 14, dadurch gekennzeichnet, daß die Außenschicht aus einem Rauheitslack (172) besteht.

16. Verpackung nach einem der Ansprüche 1 bis 15, dadurch gekennzeichnet, daß das Verpackungsmaterial der Verpackung aus Zellglas besteht.

17. Verpackung nach einem der Ansprüche 1 bis 15, dadurch gekennzeichnet, daß daß Verpackungsmaterial der Verpackung aus Kunststoff besteht.

18. Verpackung nach Anspruch 17, dadurch gekennzeichnet, daß der Kunststoff biologisch abbaubar ist.

19. Verpackung nach einem der Ansprüche 1 bis 18, dadurch gekennzeichnet, daß das Verpackungsmaterial der Verpackung mehr als 10 µm dick ist.

20. Verpackung nach Anspruch 19, dadurch gekennzeichnet, daß die Dicke des Verpackungsmaterials bis zu 20 µm beträgt.

21. Verpackung nach Anspruch 1, dadurch gekennzeichnet, daß die Schwachstellen (94) in Längsrichtung der Schwächungslinie (92) bogenförmig vertieft sind.

22. Verpackung nach Anspruch 21, dadurch gekennzeichnet, daß die Schwachstellen (94) in Längsrichtung der Schwächungslinie U-förmig vertieft sind.

23. Verpackung nach Anspruch 1, dadurch gekennzeichnet, daß die Schwächungslinie aus Paaren linearer Schwachstellen (104) von jeweils identischer Länge besteht, die parallel und nebeneinander angeordnet sind.

24. Verpackung nach Anspruch 1, dadurch gekennzeichnet, daß die Schwächungslinie (110) aus jeweils drei streifenförmigen Schwachstellen (112) gleicher Länge besteht, die parallel und nebeneinander angeordnet sind.

25. Verpackung nach Anspruch 1, dadurch gekennzeichnet, daß die Schwächungslinie (118) aus Schwachstellen (122) besteht, die jeweils entsprechend einem schraubenförmigen Muster gestaltet sind.

26. Verpackung nach Anspruch 25, dadurch gekennzeichnet, daß die Schwachstellen (122) sich in Umfangsrichtung der Schwächungslinie (118) mit ihren benachbarten Enden überlappen.

27. Verpackung nach Anspruch 1, dadurch gekennzeichnet, daß die Schwächungslinie (103) ein Muster bildet, das aus aufeinanderfolgenden, bogenförmig gekrümmten Schwachstellen (105a, 105b; 111a, 111b) besteht, welche Bögen bilden, die sich in Richtung des Vorderendes (101a) der einzelnen Verpackung (102; 107) konvex erstrecken, während die Schwachstellen (105b) jeweils in Richtung des hinteren Endes (101b; 107b) der einzelnen Verpackung (101; 107) konvex gekrümmt sind.

28. Verpackung nach Anspruch 27, dadurch gekennzeichnet, daß die bogenförmigen Schwachstellen (105a, 105b) in verhältnismäßig geringen Abständen durch Stege (105c) getrennt sind.

29. Verpackung nach Anspruch 27, dadurch gekennzeichnet, daß die bogenförmig gekrümmten Schwachstellen (111a, 111b) sich mit ihren Enden derart überlappen, daß die Enden der Schwachstellen vor und hinter einer bogenförmigen Schwachstelle in Umfangsrichtung der einzelnen Verpackung (107) angeordnet sind und in die konvexe Krümmung der Schwachstelle reichen.

30. Verpackung nach Anspruch 1, dadurch gekennzeichnet, daß die Schwächungslinie (115) aus linearen Schwachstellen (117a, 117b) besteht, die in Umfangsrichtung der einzelnen Verpackung (113) derart gegeneinander versetzt sind, daß die Enden der hintereinander angeordneten linearen Schwachstellen (117a, 117b) sich gegenseitig überlappen.

31. Verpackung nach Anspruch 1, dadurch gekennzeichnet, daß die Schwächungslinie (126) aus Schwachstellen (128) besteht, die ein Muster bilden und die in Richtung der Schwächungslinie (126) V-förmig sind.

32. Verpackung nach Anspruch 31, dadurch gekennzeichnet, daß eine Überlappung des Winkelscheitels einer V-förmigen Schwachstelle (128) durch die freien Enden der nachfolgenden V-förmigen Schwachstelle (128) vorgesehen ist.

33. Verpackung nach Anspruch 1, dadurch gekennzeichnet, daß die Außenseite des aus Zellglas hergestellten Verpackungsmaterials (170) mit einer Schicht (172) aus Rauheitslack bedeckt ist.

34. Verpackung nach Anspruch 1, dadurch gekennzeichnet, daß die Unterseite des aus Zellglas hergestellten Verpackungsmaterials mit einer luftdichten Beschichtung versehen ist.

35. Verpackung nach Anspruch 33, dadurch gekennzeichnet, daß die rauhe Struktur eines Lackes mittels normaler Teilchen hergestellt ist.

36. Verpackung nach Anspruch 33, dadurch gekennzeichnet, daß die rauhe Struktur der Zellglasoberfläche durch ein schrumpfungsartiges Trocknen des Rauheitslackes als eine Reaktion in der Trockenstufe einer Druckmaschine hergestellt ist.

37. Verpackung nach Anspruch 33, dadurch gekennzeichnet, daß die rauhe Struktur der Zellglasoberfläche durch nadelförmige Kristalle als eine Reaktion bei dem Trocknungsverfahren des Rauheitslackes hergestellt ist.

38. Verpackung nach Anspruch 33, dadurch gekennzeichnet, daß die rauhe Struktur der Zellglasoberfläche aus Kunststoffteilchen besteht, die der Lackmasse hinzugefügt sind.

39. Verpackung nach Anspruch 33, dadurch gekennzeichnet, daß eine Heißsiegelschicht auf die beiden Seiten des Zellglases aufgebracht ist.

40. Verpackung nach Anspruch 39, dadurch gekennzeichnet, daß die die Außenseite der Verpackung bildende Seite des Zellglases mit einem abdichtenden Rauheitslack versehen ist.

41. Verpackung nach Anspruch 1, dadurch gekennzeichnet, daß die Außenseite (184; 194) des Verpackungsmaterials (180; 190) für die Verpackung mindestens teilweise mit mechanischen Einprägungen (182; 183; 192; 202; 222; 232; 242; 252) und entsprechenden Vertiefungen (188; 196) auf einer Innenseite (186; 198) des Verpackungsmaterials versehen ist.

## Revendications

1. Emballage individuel substantiellement étanche à l'air (100), contenant un tampon sans applicateur pour l'hygiène féminine et dont l'extrémité arrière est munie d'une bande permettant le retrait, ledit emballage comprenant : du matériau d'emballage renfermant ledit tampon individuel et comprenant des moyens individuels disposés autour de la circonférence de l'emballage de tampon individuel (100) et permettant d'ouvrir l'emballage, caractérisé en ce que
lesdits moyens permettant d'ouvrir l'emballage sont fournis par au moins une ligne d'affaiblissement (23 ; 92 ; 102 ; 110 ; 118 ; 126 ; 132 ; 146 ; 148 ; 162 ; 176 ; 204) ; ladite ligne d'affaiblissement étant une rangée de points d'affaiblissement (22 ; 25 ; 27 ; 28 ; 36 ; 38 ; 44 ; 64 ; 66 ; 72 ; 78 ; 82 ; 86 ; 94 ; 105a : 105b ; 111b ; 112 ; 117a ; 117b ; 122 ; 128 ; 136 ; 150 ; 152 ; 178) composés de parties en relief et de parties en creux ;
le matériau d'emballage ayant une épaisseur variable le long de ces points d'affaiblissement, de telle sorte que lesdits points d'affaiblissement assurent un emballage étanche à l'air et satisfaisant du point de vue de l'hygiène pour ledit tampon et ce, jusqu'à ce que les lignes d'affaiblissement soient détruites d'une manière très silencieuse et sans laisser de résidus lorsque l'on ouvre l'emballage en agissant sur les parties (A, B) de l'emballage qui sont positionnées des deux côtés de la rangée de points d'affaiblissement.

2. Emballage selon la revendication 1, caractérisé en ce que ladite ligne d'affaiblissement est disposée autour de la circonférence de l'emballage de tampon individuel (100) à une distance d'approximativement un tiers de la longueur de l'emballage individuel (100) à partir de l'extrémité (98) de ce dernier qui est assignée à ladite extrémité arrière du tampon munie de la bande de retrait.

3. Emballage selon la revendication 1, caractérisé en ce que les points d'affaiblissement (22 ; 25 ; 27 ; 36) sont composés de creux en forme d'arc, comme montré dans une vue en coupe semi-longitudinale.

4. Emballage selon la revendication 1, caractérisé en ce que les extrémités des points d'affaiblissement (27) sont formées par des dentelures profondes et essentiellement verticales (43 ; 45), entre lesquelles s'étend une partie de fond (49) des points d'affaiblissement (27), ladite partie de fond ayant un profile longitudinal arqué.

5. Emballage selon la revendication 1, caractérisé en ce que les points d'affaiblissement (44 ; 64 ; 66) ont un profile longitudinal essentiellement en forme de U.

6. Emballage selon la revendication 1, caractérisé en ce que les points d'affaiblissement (72 ; 78) ont un profile longitudinal en forme de V.

7. Emballage selon l'une des revendications 1 à 6, caractérisé en ce que les points d'affaiblissement (36 ; 38 ; 64 ; 66) sont fournis sur le dessus (32 ; 60 ; 80) et sur le dessous (34 ; 62 ; 84) du matériau d'emballage (30 ; 58 ; 76).

8. Emballage selon la revendication 7, caractérisé en ce que les points d'affaiblissement (366 ; 38 ; 64 ; 66 ; 78 ; 82) coïncident sur le dessus (32 ; 60 ; 80) et sur le dessous (34 ; 62 ; 84).

9. Emballage selon la revendication 6 ou 7, caractérisé en ce que les points d'affaiblissement (25 ; 27) qui ont une longueur différente dans la direction de la ligne d'affaiblissement sont disposés l'un après l'autre de façon alternative.

10. Emballage selon la revendication 1, caractérisé en ce que des parties en saillie (26 ; 29 ; 40), ayant chacune la même dimension, sont disposées entre les points d'affaiblissement (22 ; 25 ; 27 ; 28 ; 36 ; 38) dans la direction et/ou dans une direction transversale à la direction de la ligne d'affaiblissement.

11. Emballage selon la revendication 1, caractérisée en ce que les points d'affaiblissement sont formés par des motifs d'affaiblissement.

12. Emballage selon l'une des revendications 1 à 11, caractérisé en ce que l'extérieur (184) du matériau d'emballage (180) est au moins partiellement muni de parties saillantes en forme de bouton (182 ; 192) afin d'améliorer la prise.

13. Emballage selon l'une des revendications 1 à 12, caractérisé en ce que le matériau d'emballage de l'emballage estmuni d'un revêtement.

14. Emballage selon la revendication 13, caractérisé en ce que le revêtement est une couche externe.

15. Emballage selon la revendication 14, caractérisé en ce que la couche externe est composée d'une laque conférant une rugosité (172).

16. Emballage selon l'une des revendications 1 à 15, caractérise en ce que le matériau d'emballage de l'emballage est composé de cellophane.

17. Emballage selon l'une des revendications 1 à 15, caractérisé en ce que le matériau d'emballage de l'emballage est composé de plastique.

18. Emballage selon la revendication 17, caractérisé en ce que le plastique est biodégradable.

19. Emballage selon l'une des revendications 1 à 18, caractérisé en ce que le matériau d'emballage de l'emballage a une épaisseur supérieure à 10 micromètres.

20. Emballage selon la revendication 19, caractérisé en ce que l'épaisseur du matériau d'emballage s'élève à 20 micromètres.

21. Emballage selon la revendication 1, caractérisé en ce que les points d'affaiblissement (94) sont en creux et arqués dans une direction longitudinale par rapport à la ligne d'affaiblissement (92).

22. Emballage selon la revendication 21, caractérisé en ce que les points d'affaiblissement (94) sont en creux et en forme de U dans la direction longitudinale par rapport à la ligne d'affaiblissement.

23. Emballage selon la revendication 1, caractérisé en ce que la ligne d'affaiblissement est constituée de paires de points d'affaiblissement linéaires (104), qui ont respectivement une longueur identique, situées parallèlement et à proximité les uns des autres.

24. Emballage selon la revendication 1, caractérisé en ce que la ligne d'affaiblissement (110) est constituée de trois points d'affaiblissement ayant respectivement la forme d'une bande (112) et étant de longueur égale, qui sont situés parallèlement et à proximité les uns des autres.

25. Emballage selon la revendication 1, caractérisé en ce que la ligne d'affaiblissement (118) est composée de point d'affaiblissement (122) qui sont respectivement conçus selon un motif ellipsoïdal.

26. Emballage selon la revendication 25, caractérisé en ce que lesdits points d'affaiblissement (122) se chevauchent au niveau de leurs extrémités adjacentes dans la direction circonférentielle par rapport à la ligne d'affaiblissement (118).

27. Emballage selon la revendication 1, caractérisé en ce que la ligne d'affaiblissement (103) forme un motif composé de points d'affaiblissement successifs, arqués et en creux (105a ; 105b ; 111a ; 111b) qui forment des arcs qui s'étendent de façon convexe dans la direction de l'extrémité avant (101a) de l'emballage individuel (101 ; 107), alors que les points d'affaiblissement (105b) sont respectivement incurvés de façon convexe en direction de l'extrémité arrière (101b ; 107b) de l'emballage individuel (101 ; 107).

28. Emballage individuel selon la revendication 27, caractérisé en ce que lesdits points d'affaiblissement arqués (105a ; 105b) sont séparés les uns des autres à des intervalles relativement petits par des parties en saillie (105c).

29. Emballage selon la revendication 27, caractérisé en ce que les points d'affaiblissement incurvés et arqués (111a ; 111b) se chevauchent au niveau de leurs extrémités de telle sorte que les extrémités des points d'affaiblissement situés devant et derrière un point d'affaiblissement arqué dans la direction circonférentielle de l'emballage individuel (107) atteignent la courbure convexe du point d'affaiblissement.

30. Emballage selon la revendication 1, caractérisé en ce que la ligne d'affaiblissement (115) est constituée de points d'affaiblissement (117a ; 117b) qui sont en retrait les uns par rapport aux autres dans la direction circonférentielle de l'emballage individuel (113), de telle sorte que les extrémités des points d'affaiblissement linéaires (117a ; 117b) situés les uns derrière les autres se chevauchent mutuellement.

31. Emballage selon la revendication 1, caractérisé en ce que la ligne d'affaiblissement (126) est constituée par des points d'affaiblissement (128) qui forment un motif et qui sont en forme de V dans la direction de la ligne d'affaiblissement (126).

32. Emballage selon la revendication 31, caractérisé en ce qu'on fournit un chevauchement du sommet de l'angle d'un point d'affaiblissement en forme de V (128) par les extrémités libres dit point d'affaiblissement en forme de V suivant (128).

33. Emballage selon la revendication 1, caractérisé en ce que l'extérieur du matériau d'emballage (170), fabriqué en cellophane, est recouvert d'une couche de laque (172) conférant une rugosité.

34. Emballage selon la revendication 1, caractérisé en ce que l'intérieur du matériau d'emballage fabriqué en cellophane est muni d'un revêtement étanche à l'air.

35. Emballage selon la revendication 33, caractérisé en ce que la structure rugueuse d'une laque est entraînée par des particules normales.

36. Emballage selon la revendication 33, caractérisé en ce que la structure rugueuse de la surface de cellophane est obtenue par un séchage ayant un effet semblable à une contraction de la laque conférant une rugosité, ce qui est une réaction qui s'opère pendant l'étape de séchage d'ans une machine d'impression.

37. Emballage selon la revendication 33, caractérisé en ce que la structure rugueuse de la surface de cellophane est fournie par des cristaux en forme d'aiguilles et qui proviennent de la réaction de la laque conférant une rugosité pendant le processus de séchage.

38. Emballage selon la revendication 33, caractérisé en ce que la structure rugueuse de la surface de la cellophane est obtenue grâce à des particules plastiques qui sont ajoutées à la masse de laque.

39. Emballage selon la revendication 33, caractérisé en ce qu'une couche thermo-scellable est appliquée sur les deux côtes de la cellophane.

40. Emballage selon la revendication 39, caractérisé en ce que le côté de la cellophane formant l'extérieur de l'emballage est muni d'une laque de scellage conférant une rugosité.

41. Emballage selon la revendication 1, caractérisé en ce que la face externe (184 ; 194) du matériau d'emballage (180 ; 190) de l'emballage est au moins partiellement munie de parties mécaniquement mises en relief (182 ; 183 ; 192 ; 202 ; 222 ; 232 ; 242 ; 252) et de parties en creux correspondantes (188 ; 196) sur une face interne (186 ; 198) dudit matériau d'emballage.
